Europäisches Patentamt

(19) European Patent Office   (11) Numéro de publication: **0 188 954**

Office européen des brevets   **B1**

(12) **FASCICULE DE BREVET EUROPÉEN**

(45) Date de publication du fascicule du brevet: 21.03.90

(21) Numéro de dépôt: 85402561.6

(22) Date de dépôt: 19.12.85

(51) Int. Cl. ⁵ : **A 61 F   5/02, A 61 F   2/44**

(54) **Dispositif d'étaiement utilisable dans une prothèse vertébrale.**

(30) Priorité: 21.12.84 FR 8419638

(43) Date de publication de la demande:
30.07.86 Bulletin 86/31

(45) Mention de la délivrance du brevet:
21.03.90 Bulletin 90/12

(84) Etats contractants désignés:
AT BE CH DE GB IT LI LU NL SE

(56) Documents cité:
US-A-4 289 123
US-A-4 401 112
US-A-4 553 273

(73) Titulaire: Daher, Youssef Hassan
9 Ter avenue de la Gaillarde
F-34000 Montpellier (FR)

(72) Inventeur: Daher, Youssef Hassan
9 Ter avenue de la Gaillarde
F-34000 Montpellier (FR)

(74) Mandataire: Bonnetat, Christian
CABINET BONNETAT 23, Rue de Léningrad
F-75008 Paris (FR)

LIBERGRAF, STOCKHOLM 1990

2

**Description**

La présente invention concerne un dispositif pour le maintien de l'écartement normal entre deux vertèbres délimitant, dans la colonne vertébrale, les extrémités d'une cavité résultant de l'élimination d'au moins une partie de vertèbre.

Il s'avère nécessaire dans un certain nombre de cas de procéder à l'ablation, au moins partielle, d'une vertèbre chez un patient. En effet, la fracture par éclatement d'une vertèbre, en particulier dans la région dorso-lombaire, peut entraîner une compression de la moelle épinière provoquant des troubles neurologiques graves, pouvant entraîner la paraplégie. De même, une tumeur vertébrale, quel que soit son niveau, peut provoquer des problèmes neurologiques similaires. Dans ces deux cas, on recourt à l'enlèvement de la vertèbre atteinte, que l'on doit bien-sûr remplacer par une prothèse. Dans le premier cas, on utilise une greffe osseuse (prothèse interne) qui va se revasculariser dans une période de temps comprise entre six et neuf mois; dans le deuxième cas, on préfère utiliser une prothèse en matière synthétique, telle que par exemple une résine.

Il est connu d'utiliser, en temps qu'armature de prothèse vertébrale, un étai constitué d'une tige filetée dont les deux extrêmités viennent se loger dans des trous forés dans les vertèbres délimitant, dans la colonne vertébrale, les extrêmités de la cavité résultant de l'élimination, au moins partielle, de la vertèbre. La tige filetée est maintenue dans ces trous par des écrous prenant appui sur les vertèbres d'extrémité. La prothèse osseuse ou synthétique, est alors placée autour de ce support.

Un dispositif de ce type présente néanmoins un certain nombre d'inconvénients. D'une part, un tel étai est relativement fragile au vu des contraintes très importantes qu'il peut subir. En outre, il est nécessaire d'utiliser une tige dont la longueur est supérieure à l'écartement entre les vertèbres d'extrémité, ce qui entraîne des difficultés de mise en place.

Certains de ces inconvénients sont évités par le dispositif décrit dans le brevet US-A-4 289 123 comportant des tiges filetées extérieures à la cavité et coopérant avec des supports fixés extérieurement sur les vertèbres d'extrémité. Cependant, l'inconvénient majeur d'un tel dispositif est de se trouver en saillie par rapport à la colonne vertébrale.

Par ailleurs, par le brevet US-A-4 401 112, on connaît un dispositif d'étaiement utilisable dans une prothèse vertébrale et comprenant des moyens d'étaiement réglables pourvus, à leurs extrémités, de moyens d'ancrage dans les vertèbres délimitant, dans la colonne vertébrale, les extrémités d'une cavité résultant de l'élimination d'au moins une partie de vertèbres. Dans ce dispositif, les moyens d'ancrage sont fixés auxdits moyens d'étaiement par une vis, cette fixation étant réalisée par le fabricant et le chirurgien n'ayant aucune action sur la solidarisation des moyens d'ancrage aux moyens d'étaiement. Après mise en place d'une seule pièce dans la cavité vertébrale par le chirurgien, les moyens d'étaiement sont écartés pour faire pénétrer les crampons dans les vertèbres d'extrémité. Une telle fixation n'est donc pas optimale.

La présente invention a donc pour but de remédier aux inconvénients des dispositifs connus en proposant un dispositif d'étaiement de prothèse vertébrale dont la mise en place est aisée, et qui est conçu de telle façon qu'il puisse résister à toutes les contraintes mécaniques qu'il est susceptible de subir.

A cette fin, selon l'invention, le dispositif pour le maintien de l'écartement normal entre deux vertèbres délimitant, dans la colonne vertébrale, les extrémités d'une cavité résultant de l'élimination d'au moins une partie de vertèbre, du type comprenant des moyens d'étaiement réglables pourvus, à leurs extrémités, de moyens d'ancrage dans lesdites vertèbres, est caractérisé en ce que les moyens d'ancrage d'au moins une extrémité desdits moyens d'étaiement (1) sont reliés de façon amovible à ladite extrémité par un emboîtement du type à tenon et mortaise.

Ainsi, la conception particulière du dispositif conforme à la présente invention est très avantageuse, notamment en ce qui concerne la facilité et la fiabilité de sa mise en place. En effet, grâce à la caractéristique de l'invention, on peut, avant l'installation des moyens d'étaiement, mettre en place les moyens d'ancrage en utilisant des moyens efficaces appropriés (marteau), ce qui permet de garantir un ancrage solide.

De plus, l'introduction du dispositif de l'invention dans la cavité entre les vertèbres est grandement facilitée, puisque l'on n'a pas à introduire, en une seule fois, l'ensemble du dispositif, mais, que l'on peut mettre en place séparément, les moyens d'ancrage, puis les moyens d'étaiement.

Enfin, le dispositif selon la présente invention est très souple en ce qui concerne le choix de l'implantation des moyens d'ancrage, une certaine tolérance étant possible dans l'alignement de ces derniers selon les positions relatives des vertèbres délimitant la cavité à étayer.

Le dispositif selon l'invention permet par ailleurs de récupérer toute la hauteur du corps vertébral enlevé, sans nécessiter d'intervention chirurgicale complémentaire. En outre, comme le dispositif ne prend pas plus de 30 % de la circonférence de la vertèbre, on peut ainsi aisément mettre en place des greffons osseux pour une consolidation naturelle.

Selon une autre caractéristique de l'invention, les moyens d'étaiement précités comprennent au moins deux éléments susceptibles d'être déplacés, parallèlement à l'axe général de la colonne vertébrale, l'un par rapport à l'autre, notamment le long de l'axe commun auxdits éléments.

En particulier, les moyens d'étaiement précités comprennent deux éléments déplaçables, parallèlement à l'axe général de la colonne vertébrale, l'un par rapport à l'autre par l'intermédiaire d'une pièce de liaison, et notamment dans ladite pièce de liaison.

La pièce de liaison précitée peut être réalisée sensiblement sous forme d'écrou, et les éléments d'étaiement précités sensiblement sous forme de tiges filetées.

Avantageusement, l'extrêmité libre d'au moins une élément d'étaiement précitée est réalisée sous forme d'un tenon pouvant s'emboîter dans une mortaise de section correspondante prévue dans l'embase précitée. Le tenon précité peut présenter une section transversale polygonale, notamment au moins sensiblement carrée.

Selon encore une autre caractéristique de l'invention, les moyens d'ancrage précités sont constitués par un certain nombre de pointes d'ancrage dont l'extrêmité inférieure présente sensiblement une forme en biseau. On peut notamment prévoir trois pointes d'ancrage disposées à environ 120° l'une par rapport à l'autre; tandis qu'une rondelle, notamment enfilée dans les pointes d'ancrage précitées, peut être prévue sous l'embase précitée.

Selon encore une autre caractéristique de l'invention, les filetages aux deux extrêmités de la pièce de liaison précitée sont de sens opposés; et on prévoit, en outre, aux deux extrêmités de la pièce de liaison précitée des moyens de blocage en position des éléments d'étaiement précités.

Enfin, on prévoit, dans la partie centrale de la pièce de liaison précitée, un certain nombre d'orifices pour l'introduction d'un moyen, tel qu'une tige, permettant de faciliter la rotation de ladite pièce.

L'invention sera mieux comprise, et d'autres détails, avantages et caractéristiques de celles-ci apparaîtront plus clairement à la lumière de la description explicative qui va suivre, faite en référence aux dessins schématiques annexés, donnés uniquement à titre d'exemple d'un mode de réalisation actuellement préféré de l'invention, et dans lesquels:

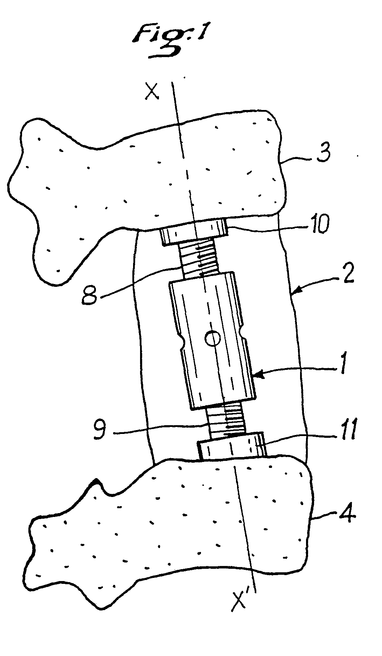

- la figure 1 est une vue schématique en perspective du dispositif selon l'invention disposé entre deux vertèbres; et:
- la figure 2 est une vue en perspective éclatée du dispositif selon l'invention, l'un des éléments d'étaiement étant représenté inséré dans la pièce de liaison, et l'autre élément hors de ladite pièce, uniquement à des fins illustratives.

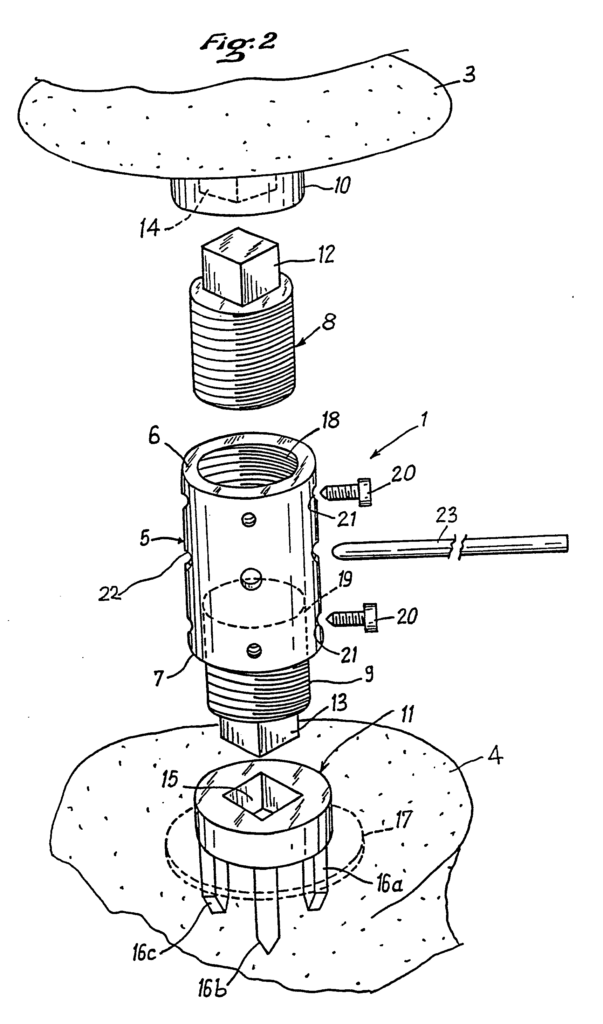

En se référant en particulier à la figure 2, le dispositif, selon l'invention, pour le maintien de l'écartement normal entre deux vertèbres 3, 4 délimitant, dans la colonne vertébrale, les extrêmités d'une cavité résultant de l'élimination d'au moins une partie de vertèbre, comprend deux embases 10, 11, munies chacune de moyens d'ancrage dans une des vertèbres d'extrêmité 3, 4; et des moyens d'étaiement 1 réglables reliant les embases 10, 11.

Les moyens d'étaiement 1 comprennent notamment deux éléments 8, 9 susceptibles, d'être déplacés, parallèlement à l'axe de la colonne vertébrale, l'un par rapport à l'autre; ils sont notamment déplaçables, le long de l'axe X, X' commun auxdits éléments, dans une pièce de liaison 5.

Selon le mode de réalisation illustré, la pièce de liaison 5 est réalisée sensiblement sous forme d'écrou, et est taraudée, au moins au voisinage de ses deux extrêmités 6, 7 de façon à recevoir les éléments d'étaiement 8, 9 précités réalisés sensiblement sous forme de tiges filetées.

L'extrêmité libre des éléments d'étaiement 8, 9, c'est-à-dire l'extrêmité en regard de l'embase 10, 11 correspondante, est susceptible d'être reliée à ladite embase correspondante par un emboîtement du type à tenon et mortaise. En particulier, elle peut être réalisée sous forme d'un tenon 12,13 pouvant s'emboîter dans une mortaise 14, 15 de section correspondante prévue dans chacune des embases 10, 11. Pour éviter le risque d'une rotation des moyens d'étaiement dans les embases, les tenons 12, 13 présentent avantageusement une section transversale polygonale, notamment carrée.

Chaque embase est munie de moyens d'ancrage constitués par un certain nombre de pointes d'ancrage 16a, 16b, 16c, dont l'extrêmité inférieure présente sensiblement une forme en biseau. Comme illustré, on peut prévoir trois pointes d'ancrage, pour chaque embase, disposées à environ 120° l'une par rapport à l'autre.

En outre, de façon à répartir les charges subies par les embases, une rondelle 17, notamment enfilée dans les pointes d'ancrage précitées, est prévue sous chaque embase.

On notera de plus que les filetages 18, 19 prévus aux deux extrêmités de la pièce de liaison 5, sont de sens opposés, tandis que l'on prévoit, aux deux extrêmités de la pièce de liaison 5, des perçages 21 dans lesquels viennent se loger des vis de blocage 20, en position, des éléments d'étaiement 8, 9.

Enfin, l'on prévoit, dans la partie centrale de la pièce de liaison 5, un certain nombre d'orifices 22 pour l'introduction d'une tige 23 permettant de faciliter la rotation de ladite pièce.

On décrira maintenant ci-après comment on peut mettre en place le dispositif d'étaiement selon l'invention.

Une cavité ayant été créée dans la colonne vertébrale d'un patient par élimination d'au moins une partie de vertèbre, suite à une fracture ou une tumeur vertébrale, on plante le embases 10, 11, munies de leurs rondelles, dans les deux vertèbres délimitant ladite cavité; on place alors, entre celles-ci, la pièce de liaison 5 dans laquelle sont vissés les éléments d'étaiement 8, 9. Par rotation de la pièce de liaison 5 (grâce notamment à l'utilisation d'une tige 23), et du fait que les deux filetages 18, 19 aux extrêmités de la pièce de liaison sont de sens opposés, on peut adapter, par un même mouvement de rotation, le niveau de la partie débordant de la pièce de liaison des deux éléments d'étaiement, à la hauteur requise (correspondant à la hauteur effective de la vertèbre manquante), tout en emboîtant le tenon

de chaque élément d'étaiement dans la mortaise de l'embase correspondante. On poursuit bien-sûr 1 rotation de la pièce de liaison 5 jusqu'au coïncement de l'ensemble du dispositif d'étaiement en position. On peut éventuellement consolider cet ensemble en utilisant les vis de blocage 20.

Une fois le dispositif d'étaiement en place, on peut noyer celui-ci dans une prothèse 2, soit osseuse, soit synthétique, de la manière usuelle.

**Revendications**

1. Dispositif pour le maintien de l'écartement normal entre deux vertèbres délimitant, dans la colonne vertébrale, les extrémités d'une cavité résultant de l'élimination d'au moins une partie de vertèbre, du type comprenant des moyens d'étaiement (1) réglables dans leur longeur pourvus, à leurs extrémités, de moyens d'ancrage (16 a, b, c) dans lesdites vertèbres, caractérisé en ce que les moyens d'ancrage (16 a, b, c) d'au moins une extrémité desdits moyens d'étaiement (1) sont reliés de façon amovible à ladite extrémité par un emboîtement du type à tenon et mortaise (12, 13, 14, 15).

2. Dispositif selon la revendication 1, caractérisé en ce qu'il comprend deux embases (10, 11) reliées par lesdits moyens d'étaiement (1), chacune étant munie de moyens d'ancrage correspondants.

3. Dispositif selon l'une des revendications 1 ou 2, caractérisé en ce que lesdits moyens d'étaiement comprennent au moins deux éléments (8, 9) susceptibles d'être déplacés, parallèlement à l'axe de la colonne vertébrale, l'un par rapport à l'autre, notamment le long de l'axe (X, X') commun auxdits éléments.

4. Dispositif selon la revendication 3, caractérisé en ce que lesdits moyens d'étaiement comprennent deux éléments déplaçables (8, 9), parallèlement à l'axe général de la colonne vertébrale, l'un par rapport à l'autre par l'intermédiaire d'une pièce de liaison (5).

5. Dispositif selon la revendication 4, caractérisé en ce que lesdits deux éléments d'étaiement (8, 9) sont susceptibles d'être déplacés, parallèlement à l'axe général de la colonne vertébrale, l'un par rapport à l'autre, dans la pièce de liaison (5).

6. Dispositif selon la revendication 5, caractérisé en ce que la pièce de liaison (5) est réalisée sensiblement sous forme d'écrou, et les éléments d'étaiement (8, 9) sensiblement sous forme de tiges filetées.

7. Dispositif selon la revendication 3 à 6, caractérisé en ce que l'extrémité libre d'au moins un élément d'étaiment (8, 9) est réalisée sous forme d'un tenon (12, 13) pouvant s'emboîter dans une mortaise (14, 15) de section correspondante prévue dans ladite embase (10, 11).

8. Dispositif selon la revendication 7, caractérisé en ce que le tenon (12, 13) présente une section transversale polygonale.

9. Dispositif selon la revendication 8, caractérisé en ce que le tenon (12, 13) présente une section transversale au moins sensiblement carrée.

10. Dispositif selon l'une quelconque des revendications 1 à 9, caractérisé en ce que lesdits moyens d'ancrage sont constitués par un certain nombre de pointes d'ancrage (16a), (16b), (16c) dont l'extrémité inférieure présente sensiblement une forme en biseau.

11. Dispositif selon la revendication 10, caractérisé en ce qu'on prévoit trois pointes d'ancrage (16a, 16b, 16c) disposées à environ 120° l'une par rapport à l'autre.

12. Dispositif selon l'une quelconque des revendications 2 à 11, caractérisé en ce qu'une rondelle (17), notamment enfilée dans les pointes d'ancrage (16a), (16b), (16c), est prévue sous chaque embase.

13. Dispositif selon l'une quelconque des revendications 6 à 12, caractérisé en ce que les filetages (18, 19) prévus aux deux extrémités de la pièce de liaison (5) sont de sens opposés.

14. Dispositif selon l'une quelconque des revendications 5 à 13, caractérisé en ce que l'on prévoit, aux deux extrémités de la pièce de liaison (5), des moyens (20, 21) de blocage en position des éléments d'étaiement (8, 9).

15. Dispositif selon l'une quelconque des revendications 5 à 14, caractérisé en ce que l'on prévoit, dans la partie centrale de la pièce de liaison (5), un certain nombre d'orifices (22) pour l'introduction d'un moyen, tel qu'une tige (23), permettant de faciliter la rotation de ladite pièce.

**Patentansprüche**

1. Vorrichtung zum Aufrechterhalten des normalen Abstandes zwischen zwei Wirbeln in der Wirbelsäule, die die Enden eines Hohlraumes begrenzen, der sich als Folge der Herausnahme zumindest eines Wirbelteiles ergibt, und bestehend aus in der Länge regelbaren Mitteln der Absteifung (1), die an ihren Enden mit Mitteln der Verankerung (16a, b, c) in den Wirbeln versehen sind, *dadurch gekennzeichnet*, daß die Mittel der Verankerung (16a, b, c) zumindest eines Endes der Mittel der Absteifung (1) durch eine Einfügung in der Art eines Zapfens und Zapfenloches (12, 13, 14, 15) lösbar mit dem Ende verbunden sind.

2. Vorrichtung nach Anspruch 1 *dadurch ge-*

kennzeichnet, daß sie zwei durch die Mittel der Absteifung (1) verbundene Sitz- oder Befestigungsflächen (10, 11) aufweist, von denen jede mit entsprechenden Mitteln der Verankerung ausgerüstet ist.

3. Vorrichtung nach einem der vorhergehenden Ansprüche 1 oder 2, dadurch gekennzeichnet, daß die Mittel der Absteifung zumindest zwei Glieder (8, 9) aufweisen, die parallel zur Achse der Wirbelsäule, insbesondere längs der den Gliedern gemeinsamen Achse (X, X) gegenseitig verschiebbar sind.

4. Vorrichtung nach Anspruch 3, dadurch gekennzeichnet, daß die Mittel der Absteifung zwei parallel zur allgemeinen Achse der Wirbelsäule mittels eines Verbindungsteiles (5) gegenseitig verschiebbare Glieder (8, 9) aufweisen.

5. Vorrichtung nach Anspruch 4, dadurch gekennzeichnet, daß die beiden Absteifungslieder (8, 9) parallel zur allgemeinen Wirbelsäule in dem Verbindungsteil (5) gegenseitig verschoben werden können.

6. Vorrichtung nach Anspruch 5, dadurch gekennzeichnet, daß das Verbindungsteil (5) allgemein in Form einer Schraubermutter und die Absteifungsglieder (8, 9) allgemein in Form von Schraubenspindeln ausgebildet sind.

7. Vorrichtung nach einem der vorhergehenden Ansprüche 3 bis 6, dadurch gekennzeichnet, daß das freie Ende zumindest eines Absteifungsgliedes (8, 9) in Form eines Zapfens (12, 13) ausgebildet ist, der sich in einem in der Befestigungsfläche (10, 11) vorgesehenen Zapfenloch (14, 15) entsprechenden Querschnitts einfügen läßt.

8. Vorrichtung nach Anspruch 7, dadurch gekennzeichnet, daß der Zapfen (12, 13) ein polygonales Querprofil hat.

9. Vorrichtung nach Anspruch 8, dadurch gekennzeichnet, daß der Zapfen (12, 13) ein zumindest allgemein quadratisches Querprofil hat.

10. Vorrichtung nach einem der vorhergehenden Ansprüche 1 bis 9, dadurch gekennzeichnet, daß die Mittel der Verankerung aus einer bestimmten Anzahl von Verankerungsspitzen (16a), (16b), (16c) bestehen, deren unteres Ende allgemein die Form einer abgefasten Kante aufweist.

11. Vorrichtung nach Anspruch 10, dadurch gekennzeichnet, daß drei Verankerungsspitzen (16a, 16b, 16c) vorgesehen sind, die um etwa 120° gegenseitig versetzt angeordnet sind.

12. Vorrichtung nach einem der vorhergehenden Ansprüche 2 bis 11, dadurch gekennzeichnet, daß unter jeder Befestigungsfläche eine in die Verankerungsspitzen (16a, 16b, 16c) eingefädelte Unterlegscheibe (17) vorgesehen ist.

13. Vorrichtung nach einem der vorhergehenden Ansprüche 6 bis 12, dadurch gekennzeichnet, daß die auf den beiden Enden des Verbindungteiles (5) vorgesehenen Gewinde (18, 19) gegenläufig sind.

14. Vorrichtung nach einem der vorhergehenden Ansprüche 5 bis 13. dadurch gekennzeichnet, daß an den beiden Enden des Verbindungsteiles (5) Blockiermittel (20, 21) vorgesehen sind, die zum feststellen der Position der Absteifungsglieder (8, 9) dienen.

15. Vorrichtung nach einem der vorhergehenden Ansprüche 5 bis 14, dadurch gekennzeichnet, daß in dem mittleren Teil des Verbindungsteiles (5) eine bestimmte Anzahl von Öffnungen (22) zum Einführen eines Mittels wie einer Spindel (23) vorgesehen sind, durch die die Drehung des Teiles erleichtert wird.

**Claims**

1. Device for maintaining the normal spacing between two vertebrae defining, in the vertebral column, the ends of a cavity resulting from the removal of at least a part of a vertebra, of the type comprising buttressing means (1) having an adjustable length and provided, at their ends, with means for anchoring (16a, b, c) in said vertebrae, characterized in that the anchoring means (16a, b, c) of at least one end of said buttressing means (1) are connected removably to said end by a mortice and tenon type fitting (12, 13, 14, 15).

2. Device according to claim 1, characterized in that it comprises two bases (10, 11) connected together by said buttressing means (1), each one having corresponding anchoring means.

3. Device according to one of claims 1 or 2, characterized in that said buttressing means comprise at least two elements (8, 9) able to be moved with respect to each other parallel to the axis of the vertebral column; more particularly along the axis (X, X') common to said elements.

4. Device according to claim 3, characterized in that said buttressing means comprise two elements (8, 9) mowable with respect to each other parallel to the general axis of the vertebral column by means of a connecting piece (5).

5. Device according to claim 4, characterized in that said two buttressing elements (8, 9) are able to be moved with respect to each other parallel to the general axis of the vertebral column in the connecting piece (5).

6. Device according to claim 5, characterized in that said connecting piece (5) is substantially in the form of a nut and the buttressing elements (8, 9) substantially in the form of threaded rods.

9

7. Device according to claims 3 to 6, characterized in that the free end of at least one buttressing element (8, 9) is in the form of a tenon (12, 13) which may fit into a mortice (14, 15) of corresponding section provided in said base (10, 11).

8. Device according to claim 7, characterized in that the tenon (12, 13) has a polygonal cross-section.

9. Device according to claim 8, characterized in that the tenon (12, 13) has a cross-section at least substantially square.

10. Device according to any one of claims 1 to 9, characterized in that said anchoring means are formed by a certain number of anchoring points (16a, 16b, 16c) whose lower end has substantially a bevelled form.

11. Device according to claim 10, characterized in that three anchoring points (16a, 16b, 16c) are provided disposed at about 120° with respect to each other.

12. Device according to any one of claims 2 to 11, characterized in that a washer (17) is provided under each base more particularly fitted in the anchoring points (16a, 16b, 16c).

13. Device according to any one of claims 6 to 12, characterized in that the threads (18, 19) provided at the two ends of the connecting piece (5) are of opposite directions.

14. Device according to any one of claims 5 to 13, characterized in that means (20, 21) are provided at both ends of the connecting piece (5) for locking the buttressing elements (8, 9) in position.

15. Device according to any one of claims 5 to 14, characterized in that a certain number of orifices (22) are provided in the central part of the connecting piece (5) for introducing a means, such as a rod (23), for facilitating rotation of said piece.

*Fig.1*

Fig.2